# EUROPÄISCHE PATENTSCHRIFT

(11) **EP 0 611 757 B1**
(45) Veröffentlichungstag und Bekanntmachung des Hinweises auf die Patenterteilung: **20.01.1999**
(21) Anmeldenummer: 94101671.9
(22) Anmeldetag: 04.02.1994
(51) Int. Cl.: C07D 213/74, C07D 513/04

(54) **Verfahren zur Herstellung von Azaphenothiazinen**
Process for the preparation of azaphenothiazines
Procédé de préparation d'azaphénothiazines

(30) Priorität: 19.02.1993 DE 4305080
(43) Veröffentlichungstag der Anmeldung: 24.08.1994
(73) Patentinhaber: ASTA Medica Aktiengesellschaft, D-01277 Dresden (DE)
(72) Erfinder: Kutscher, Bernhard Dr., D-63477 Maintal (DE); Dieter, Reinhold Dr., D-64291 Darmstadt (DE)
(74) Vertreter: Decker, Karl-Ludwig

(56) Entgegenhaltungen:
- EP-A- 0 174 458
- DE-C- 964 050
- FR-A- 1 170 119
- FR-A- 1 268 454
- FR-A- 2 581 067
- GB-A- 961 385

## Beschreibung

Die vorliegende Erfindung betrifft ein neues, verbessertes Verfahren zur Herstellung von Azaphenothiazinen, vorzugsweise Pyrido(3,2-b)benzothiazinen und deren Salzen. Azaphenothiazin dient als wertvolle Vorstufe für pharmazeutische Wirkstoffe wie Pipazetat (INN) (Selvigon(R)), Isothipendyl (INN) (Andantol(R)) und Prothipendyl (INN) (Dominal(R)). Die Synthese der Verbindung wird in der Patentschrift DE-PS 964050 sowie J. Org.Chem. 24, S.1156-1157, 1959, ausgehend von 2- Phenylaminopyridin und elementarem Schwefel durch Cyclisierung in Schmelze unter Iodkatalyse beschrieben. Die Entstehung von 1 Mol H₂S und die im Unterschied zu Benzothiazinen (Phenothiazinen) notwendigen hohen Reaktionstemperaturen von 230°C - 250°C sowie die aufwendige Aufarbeitungsprozedur sind aus heutiger Sicht als nachteilig anzusehen. Die in der oben zitierten deutschen Patentschrift unter milderen Bedingungen eingesetzten Beschwefelungsreagenzien SCl₂ und S₂Cl₂ führen bei Nacharbeitung der Patentschrift zu unbefriedigenden Produktmengen. Neben diesem einstufigen Verfahren ausgehend von 2-Phenylaminopyridin sind mit der Arbeit von H.L.Yale und E.Sowinski, J. Org. Chem., S.1651 (1958) und der französischen Anmeldung FR Nr. 1.170.119 mehrstufige Verfahren bekannt, die entweder von 2-Chlor-3-amino-pyridin oder 2-Chlor-3-nitropyridin und 2-Aminothiophenol (2- Mercaptoanilin) (sog. Smiles-Umlagerung, N.L. Smiles, J. Org. Chem. 15, 1125 (1950) und R. R. Gystra (Ed.): Phenothiazines and 1,4-Benzothiazines-Chemical and Biomedical Aspects, Elsevier, Amsterdam 1988, (USP 2943086) oder 2-Chlor-3-mercaptopyridin und 2-Chlor-nitrobenzol ausgehen.Beide Verfahren konnten aus ökonomischen Gründen nicht in den technischen Maßstab überführt werden.

In all den genannten Verfahren wird die Zielverbindung als Base im Vakuum destillativ und zusätzlich durch Salzbindung gereinigt.

Dieses Reinigungsverfahren belastet das Produkt thermisch und führt zu Rückständen, die mangels weiterer Verwertungsmöglichkeiten als Sondermüll entsorgt werden müssen. Reinigung über Salzbildung erfordert einen weiteren zusätzlichen Verfahrensschritt und läßt den Aufwand steigen.

Es besteht also der Bedarf nach einem Verfahren, das die geschilderten Nachteile des Standes der Technik vermeidet und das darüberhinaus den in der Zwischenzeit gestiegenen Anforderungen an die Umweltverträglichkeit von Produktionsverfahren durch Vermeidung von Nebenprodukten und hohen Reaktionstemperaturen genügt. Darüberhinaus ist es vorteilhaft, einige Verfahrensschritte einzusparen.

Überraschenderweise wurde gefunden, daß man 2-Chlorpyridine der allgemeinen Formel I 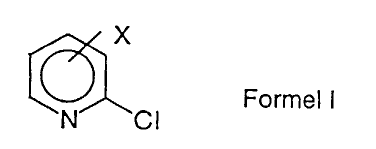 wobei gilt:
X hat die Bedeutung von Wasserstoff, Alkylgruppen mit der Kettenlänge von 1-6 Kohlenstoffatomen, wie beispielsweise die Methylgruppe, die Ethylgruppe,die Propylgruppe, die Isopropylgruppe, die Butylgruppe, die Isobutylgruppe und die tert.-Butylgruppe, durch ein oder mehrere Halogenatome substituierte Alkylgruppen, Carboxy mit 1-5 Kohlenstoffatomen und wobei die genannten Gruppen einmal oder mehrfach in den 4-, 5-, oder 6-Positionen des 2-Chlor-pyridingrundkörpers vorkommen können,
mit 2-Mercaptanilinen der allgemeinen Formel II 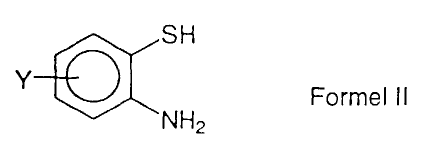 wobei gilt:
Y hat die Bedeutung von Wasserstoff, Alkylgruppen mit der Kettenlänge von 1-6 Kohlenstoffatomen, wie beispielsweise die Methylgruppe und die Ethylgruppe,die Propylgruppe, die Isopropylgruppe, die Butylgruppe, die Isobutylgruppe und die tert.-Butylgruppe, durch ein oder mehrere Halogenatome substituierte Alkylgruppen, Halogene, wie beispielsweise Fluorid, Chlorid und Bromid, Alkoxy mit 1-5 Kohlenstoffatomen, Carboxy mit 1-5 Kohlenstoffatomen, wobei die genannten Gruppen einmal oder mehrfach in den 4-, 5- oder 6- Positionen des 2-Mercaptoanilingrundkörpers vorkommen können, in Gegenwart katalytischer oder
stöchiometrischer Mengen geeigneter Oxidationsmittel, beispielsweise Persäuren, insbesondere Perameisensäure, Peressigsäure oder Perbenzoesäure, H₂O₂, Fe³⁺-Salze, V/Mo/Ag-Oxide, Peroxide, Th(OCOCF₃)₃, vorzugsweise Iod sowohl in Substanz als auch in indifferenten hochsiedenden Lösungsmitteln oder in Gegenwart von Radikalstartern wie Azabisbutyronitril Di- tert.-Butylperoxid oder Benzolyperoxid umsetzt.
Als indifferente hochsiedende Lösungsmittel kommen in Frage:
V-Methylpyrrolidon, Sulfolan, Diphenylether, Diphenyl, Diphyl. Die Temperatur liegt bei 170°C - 210°C, vorzugsweise 190°C - 200°C.
Die Isolierung der 2-(2-Mercaptophenylamino)pyridin-Zwischenstufe ist möglich, aber nicht unbedingt erforderlich. 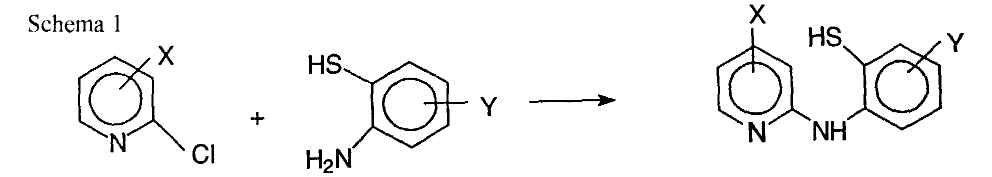 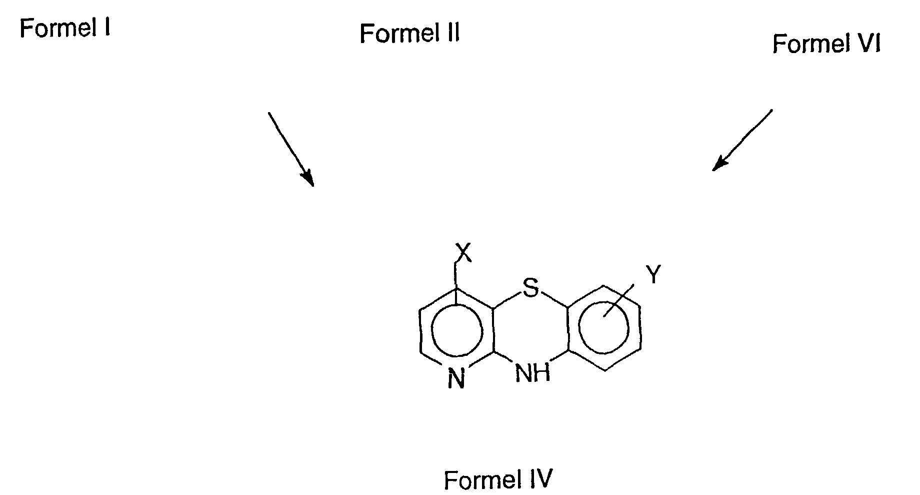 Die Verbindungen mit der allgemeinen Formel VI (Bedeutungen der Reste X und Y wie vorstehend) können isoliert werden oder werden ohne Isolierung weiter zu Verbindungen der allgemeinen Formel IV (Bedeutungen der Reste X und Y wie vorstehend) umgesetzt.

Das Verfahren zeichnet sich darüber hinaus dadurch aus, daß die entsprechenden Azaphenothiazinium-Salze durch einfaches Eintragen des Reaktionsgemisches in verdünnte wässrige Säuren, beispielsweise Mineralsäuren, vorzugsweise verdünnte Salzsäure, in sehr guter Qualität direkt auskristallisiert werden können; die Freisetzung der Azaphenothiazine aus ihren Salzen gelingt auf üblichem Wege durch Basenbehandlung. Als Basen kommen beispielsweise Alkalhydroxide in Frage, vorzugsweise werden verdünnte NaOH oder konzentrierter Ammoniak verwendet.

Weitere Vorteile des Verfahrens sind:
1. Deutlich verkürzte Reaktionszeiten
2. Auf einen Zusatz der nach Patent DBP 964050 erforderlichen Nickel- und Eisenkatalysatoren kann verzichtet werden.
Diese Eisen-oder Nickelkatalysatoren stellen eine Abwasserbelastung dar, die durch das erfindungsgemäße Verfahren vermieden wird.

Das neue Verfahren mit den verschiedenen Varianten wird durch die nachfolgenden Beispiele belegt. Die als Zwischenstufe isolierte oder direkt in situ generierte und weiter umgesetzte Verbindung 2-(2-Mercaptophenylamino)pyridin (MAP), siehe Beispiel 2 (Formel V) 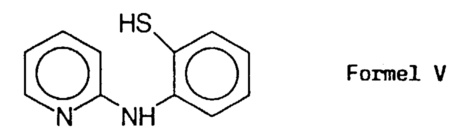 und das entsprechende Dimere (Formel VII) wurden in der Literatur bisher nicht beschrieben.

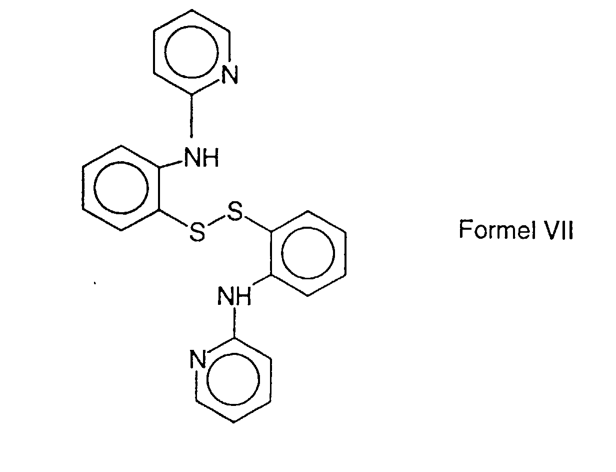

Das folgende Fließschema verdeutlicht noch einmal beispielhaft die erfindungsgemäße Reaktion.

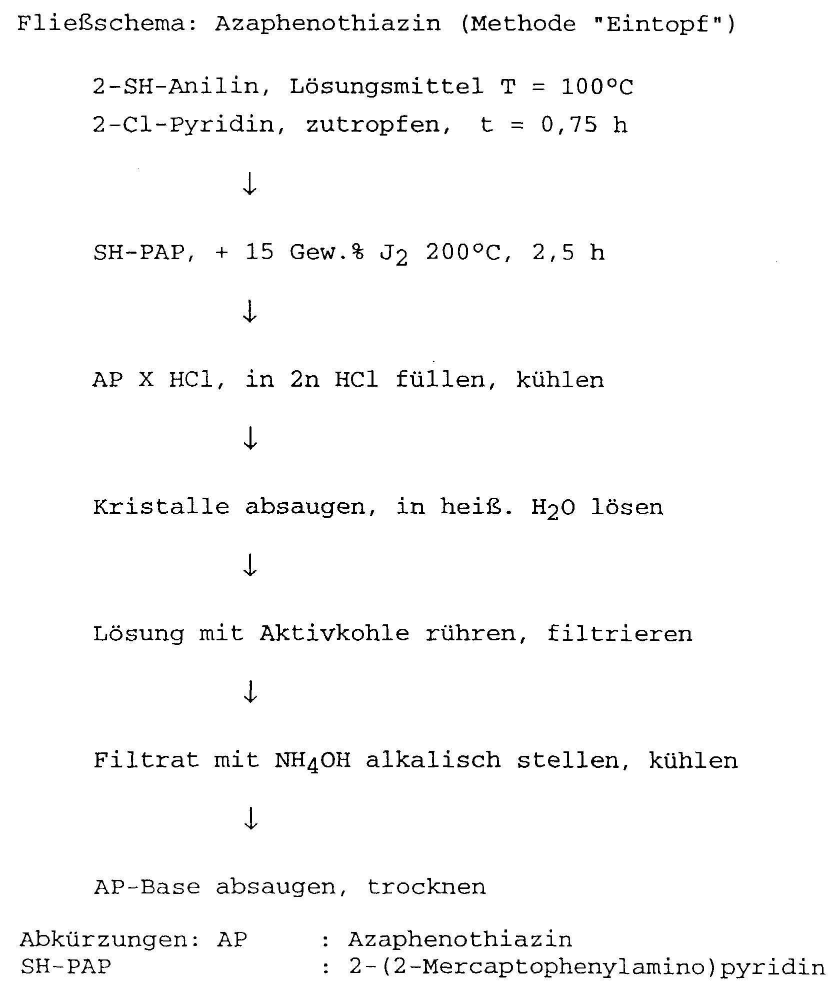

### Beispiel 1:

### Synthese von Azaphenothiazin (AP)

0.4 mol 2-Mercaptoanilin (MW = 125,2) werden in 80 ml Diphyl (25 % Diphenyl / 75 % Diphenylether) vorgelegt und bei 90°C-125°C 0,4 mol 2-Chlorpyridin (MW = 113,6) zugetropft. Dabei erfolgt ein exothermer Übergang von Suspension zu Emulsion. Anschließend werden 0.11 Äquivalente (14,3 g =15 Gew.%, bezogen auf umgesetztes 2-Mercaptoanilin) Iod zugegeben, 3 Stunden bei 200°C gerührt, auf 100°C gekühlt, 200 ml 2 nHCl zugegeben und die Phasen getrennt.Die obere Schicht (Diphyl) wird abgetrennt und kann nach Trocknung mit Na₂SO₄ wieder eingesetzt werden. Aus der unteren, wässrigen Schicht kristallisiert nach Abkühlen Azaphenothiazin-Hydrochlorid aus. Das Produkt wird abgesaugt, in 300 ml H₂O gelöst, mit 10 g Aktivkohle gerührt. Nach Abfiltrieren der Aktivkohle wird das Filtrat mit 25 %iger NaOH basisch gestellt (pH 9-11). Die ausfallende AP-Base wird abgesaugt und bei 60°C getrocknet.

AP-Base-Ausbeute: 30,4 g (58%) Schmelzpunkt: 114°C-116°C

| Elementaranalyse: | | | | |
|---|---|---|---|---|
| C | ber. | 65.97 | gef. | 66.22 |
| H | ber. | 4.03 | gef. | 3.96 |
| N | ber. | 13.99 | gef. | 13.92 |

### Beispiel 2:

### Synthese von 2-(2-Mercaptophenylamino)-pyridin (MAP)

Zu einer siedenden Lösung von 375 g (3 mol)
2- Mercaptoanilin in 1,8 1 2-Propanol tropft man unter Rühren und Schutzgasatmosphäre innerhalb von 15 min 340,5 g (3 mol) 2-Chlorpyridin. Nach beendeter Zugabe rührt man 5 Stunden bei Siedetemperatur nach. Anschließend läßt man auf Raumtemperatur abkühlen, filtriert das ausgefallene gelbliche Produkt ab und wäscht zweimal mit je 50 ml 2-Propanol nach. Nach dem Trocknen im Vakuum erhält man 592 g (86 % d. Th.)2-(2-Mercaptophenylamino)-pyridin als farblose bis gelbliche Kristalle.

Schmelzintervall 152°C- 162°C.

| Elementaranalyse: | | | | | | |
|---|---|---|---|---|---|---|
| Ber.: | C: | 55,34 | H: | 4,64 | N: | 11,73 |
| Gef.: | C: | 55,60 | H: | 4,69 | N: | 11,72 |

### Beispiel 3:

### Synthese von Azaphenothiazin (Pyrido-[3,2-b][1,4]benzothiazin = AP)

### Variante A

Eine Suspension aus 50 g 2-(2-Mercaptophenylamino)-pyridin, 8 g Iod und 200 ml Sulfolan wird unter Rühren 3 h auf 200°C erhitzt. Anschließend wird das Sulfolan im Vakuum abdestilliert. Der Rückstand wird mit 150 ml n-Butanol versetzt und nach Zusatz von 100 ml 10 %iger Natronlauge 1 Stunde bei 80°C gerührt. Nach Abkühlen wird die alkoholische Phase abgetrennt und im Vakuum eingeengt. Der Rückstand wird in 100 ml 2 N Salzsäure aufgenommen und nach Zugabe von 5 g Aktivkohle 1 Stunde bei 90°C-95°C intensiv gerührt. Anschließend filtriert man von unlöslichen Bestandteilen ab und läßt unter Rühren bei 0°C-5°C das Thiopyram-Hydrochlorid kristallisieren. Die orangegelben Kristalle werden abfiltriert und zweimal mit je 20 ml Wasser nachgewaschen. Das Produkt wird daraufhin mit 50 ml Wasser versetzt. Zu der resultierenden Mischung tropft man bei 80°C-85°C 15 ml konzentriertes Ammoniak zu. Nach beendetem Zutropfen läßt man auf Raumtemperatur abkühlen und rührt 30 min nach. Die ausgefallene Thiopyram-Base wird abgesaugt, zweimal mit je 10 ml Wasser gewaschen und im Vakuum getrocknet. Man erhält 17,7 g (42% d. Th.) Thiopyram (= AP).

Schmelzpunkt: 115°C-116°C

| Elementaranalyse: | | | | | | |
|---|---|---|---|---|---|---|
| ber.: | C: | 65,97 | H: | 4,03 | N: | 13,99 |
| gef.: | C: | 65,43 | H: | 4.03 | N: | 13,96 |

### Variante B

Eine Suspension aus 50 g 2-(2-Mercaptophenylamino)pyridin, 7,5 g Iod und 100 ml Diphyl wird unter Rühren 2,5 Stunden auf 200°C-205°C erhitzt. Nach Abkühlen auf 80°C wird das Reaktionsgemisch mit 100 ml 2 N Salzsäure versetzt und 15 min bei 80°C-85°C gerührt. Anschließend werden die Phasen getrennt. Aus der wässrigen Schicht kristallisiert nach Abkühlen Thiopyramhydrochlorid aus. Die orange-gelben Kristalle werden abfiltriert und zweimal mit je 20 ml Wasser nachgewaschen. Das Produkt wird daraufhin mit 150 ml Wasser versetzt. Zu der resultierenden Mischung tropft man bei 80°C-85°C 15 ml konzentriertes Ammoniak. Nach beendetem Zutropfen läßt man auf Raumtemperatur abkühlen und rührt 30 min nach. Die ausgefallene Thiopyram-Base wird abgesaugt, zweimal mit je 10 ml Wasser gewaschen und im Vakuum getrocknet. Man erhält 18,9 g AP-Base (45,1 % d. Th.)

Schmelzpunkt 114°C - 116°C

| Elementaranalyse: | | | | | | |
|---|---|---|---|---|---|---|
| Ber.: | C: | 65,97 | H: | 4,03 | N: | 13,99 |
| Gef.: | C: | 65,27 | H: | 4,02 | N: | 13,93 |

### Variante C

Eine Mischung aus 50 g 2-(2-Mercaptophenylamino)-pyridin und 7,5 g Iod werden unter Rühren 2 Stunden auf 200°C - 205°C erhitzt. Nachdem Abkühlen wird die Schmelze mit 30 ml 10 %ige Salzsäure und 5 g Aktivkohle versetzt und 1 Stunde bei ca. 70°C gerührt. Anschließend filtriert man von unlöslichen Bestandteile ab und läßt unter Rühren bei 0°C-5°C das Thiopyramhydrochlorid kristallisieren. Die orangegelben Kristalle werden abfiltriert und zweimal mit je 10 ml Wasser gewaschen und im Vakuum getrocknet. Man erhält 14,5 g (34,7 % d. Th.) AP.

Schmelzpunkt 106°C-110°C

| Elementaranalyse: | | | | | | |
|---|---|---|---|---|---|---|
| ber.: | C: | 65,97 | H: | 4,03 | N: | 13,99 |
| gef.: | C: | 66,06 | H: | 4,13 | N: | 14,24 |

### Variante D

Zu einer siedenden Lösung von 31,3 g 2-Mercaptoanilin in 150 ml 2-Propanol tropft man unter Rühren und Schutzgasatmosphäre innerhalb von 15 min 28,4 g 2-Chlorpyridin zu. Nach beendeter Zugabe rührt man 7 h bei Siedetemperatur nach. Anschließend wird das Isopropanol abdestilliert. Zu dem Rückstand werden 9 g Iod dazugegeben und unter Rühren 1,5 Stunden bei 200°C-205°C erhitzt. Die Schmelze wird mit 150 ml n-Butanol versetzt und nach Zusatz von 100 ml 10%iger Natronlauge 1 Stunde bei 80°C gerührt. Nach Abkühlen wird die alkoholische Phase abgetrennt und im Vakuum eingeengt. Der Rückstand wird in 100 ml 2 N Salzsäure aufgenommen und nach Zugabe von 5 g Aktivkohle 1 Stunde bei 90°C-95°C intensiv gerührt. Anschließend filtriert man von unlöslichen Bestandteilen ab und läßt unter Rühren bei 0°C-5°C das Thiopyramhydrochlorid kristallisieren.Die orangegelben Kristalle werden abfiltriert und zweimal mit je 20 ml Wasser nachgewaschen. Das Produkt wird daraufhin mit 150 ml Wasser versetzt. Zu der resultierenden Mischung tropft man bei 80°C-85°C 15 ml konzentriertes Ammoniak zu. Nach beendetem Zutropfen läßt man auf Raumtemperatur abkühlen und rührt 30 min nach. Die ausgefallene Thiopyram-Base wird abgesaugt, zweimal mit je 10 ml Wasser gewaschen und im Vakuum getrocknet. Man erhält 8,9 g (38 % d. Th.) Thiopyram.

Schmelzpunkt: 115°C - 116°C

| Elementaranalyse: | | | | | | |
|---|---|---|---|---|---|---|
| ber.: | C: | 65,97 | H: | 4,03 | N: | 13,99 |
| gef.: | C: | 65,89 | H: | 4,04 | N: | 14,09 |

Die Verbindungen gemäß Beispiel 4 - 6 wurden nach der Vorschrift in Beispiel 1 unter Verwendung der geeigneten Vorstufen erhalten.

### Beispiel 4:

### 1-Aza-8-trifluormethyl-phenothiazin-hydrochlorid

Molekulargewicht: 304,63
Schmelzpunkt: 234°C

| Elementaranalyse: | | | | | | |
|---|---|---|---|---|---|---|
| Ber.: | C: | 47,30, | H: | 2,65, | N: | 9,19 |
| gef.: | C | 47,05, | H: | 2,48; | N: | 8,79 |

### Beispiel 5:

1 Aza-2-methyl-phenothiazin
Molekulargewicht 305,13
Schmelzpunkt: 186°C

| Elementaranalyse: | | | | | | |
|---|---|---|---|---|---|---|
| ber.: | C: | 47,23, | H: | 4,62, | N: | 9,18 |
| gef.: | C | 47,28, | H: | 3,62, | N: | 9,21 |

### Beispiel 6:

1-Aza-3-carboxy-phenothiazin (D 22571)
Molekulargewicht: 244,18
Schmelzpunkt : >300°C

| Elementaranalyse: | | | | | | |
|---|---|---|---|---|---|---|
| ber.: | C: | 59,00 | H: | 3,30 | N: | 11,47 |
| gef.: | C: | 57,85 | H: | 3,23 | N: | 11,07 |

## Patentansprüche

1. 2-(2-Mercaptophenylamino)pyridin der Formel V

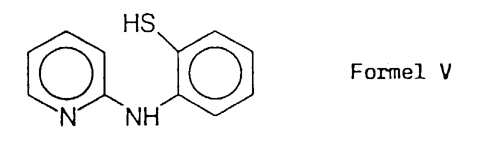

2. Verfahren zur Herstellung von 2-(2-Mercaptophenylamino)pyridin der Formel V, 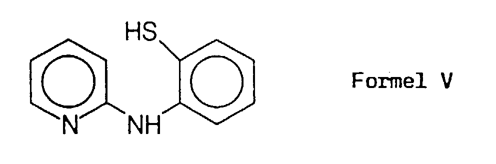 dadurch gekennzeichnet, daß man eine Verbindung der allgemeinen Formel I, 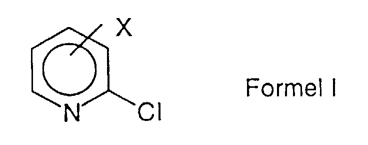 wobei X die Bedeutung von Wasserstoff hat, mit einer Verbindung der allgemeinen Formel II, 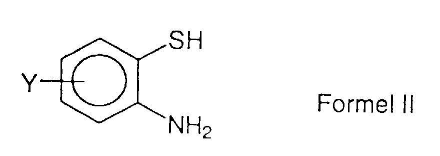 wobei Y die Bedeutung von Wasserstoff hat, zur Verbindung gemäß Anspruch 1 umsetzt.

3. Verfahren zur Herstellung von Azaphenothiazinen der allgemeinen Formel IV, 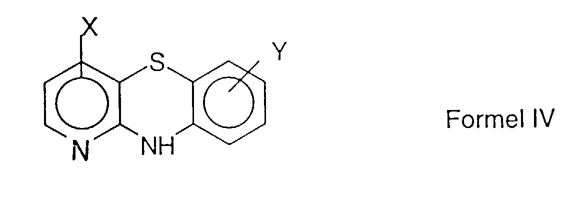 wobei X die Bedeutung von Wasserstoff, Alkylgruppen mit der Kettenlänge von 1-6 Kohlenstoffatomen, die durch ein oder mehrere Halogenatome substituiert sein können oder Carboxy mit 1-5 Kohlenstoffatomen haben kann und wobei die genannten Gruppen einmal oder mehrfach in den 4-, 5- oder 6- Positionen des 2-Chlorpyridingrundkörpers vorkommen können, und wobei Y die Bedeutung von Wasserstoff, Alkylgruppen mit der Kettenlänge von 1-6 Kohlenstoffatomen, die durch ein oder mehrere Halogenatome substituiert sein können, Halogene, Alkoxy mit 1-5 Kohlenstoffatomen oder Carboxy mit 1-5 Kohlenstoffatomen haben kann und wobei die genannten Gruppen einmal oder mehrfach in den 4-, 5- oder 6- Positionen des 2-Mercaptoanilingrundkörpers vorkommen können, dadurch gekennzeichnet, daß man Verbindungen der allgemeinen Formel I 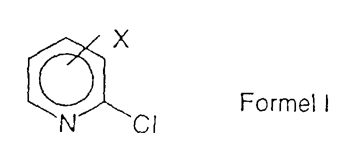 wobei X die genannte Bedeutung hat,
mit Verbindungen der allgemeinen Formel II 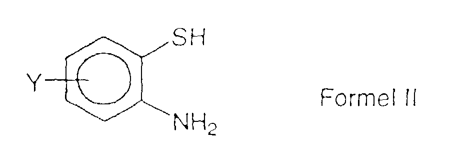 wobei Y die genannte Bedeutung hat,
in Gegenwart von Oxidationsmitteln in inerten Lösungsmitteln umsetzt

4. Verfahren gemäß Anspruch 3, dadurch gekennzeichnet, daß man als Oxidationsmittel lod, Eisen Eisen(III)-Salze, MnO₂, Peroxide, Persäuren, H₂O₂, V/Mo/Ag-Oxide oder Radikalstarter benutzt.

5. Verfahren gemäß Anspruch 3, worin x die Methylgruppe, die Ethylgruppe, die Propylgruppe, die Isopropylgruppe, die Butylgruppe, die Isobutylgruppe oder die tert. Butylgruppe und y die Methylgruppe, die Ethylgruppe, die Propylgruppe, die Isopropylgruppe, die gruppe, die Ethylgruppe, die Propylgruppe, die Isopropylgruppe, die Butylgruppe, die Isobutylgruppe, die tert. Butylgruppe, Fluorid, chlorid oder Bromid bedeutet.

## Claims

1. 2-(2-Mercaptophenylamino)pyridine of the formula V

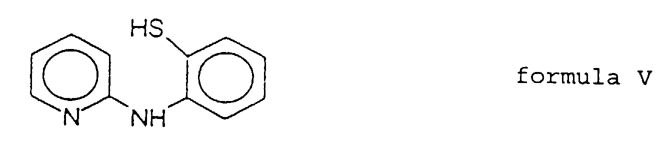

2. Process for the production of 2-(2-mercaptophenylamino)pyridine of the formula V 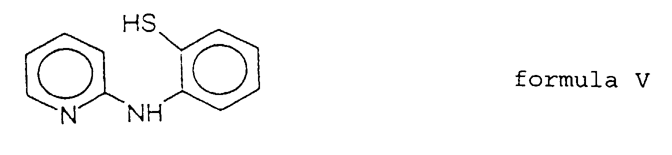 characterised in that a compound of the general formula I 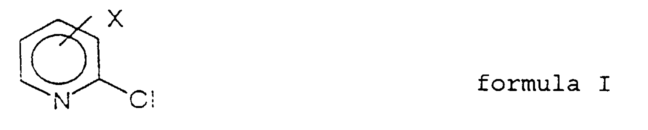 wherein X means hydrogen, is reacted with a compound of the general formula II, 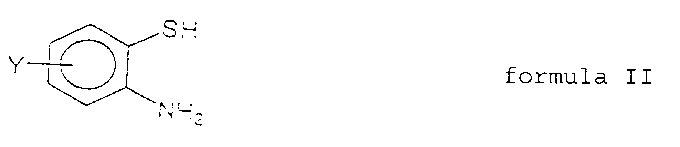 wherein Y means hydrogen, to yield the compound according to claim 1.

3. Process for the production of azaphenothiazines of the general formula IV, 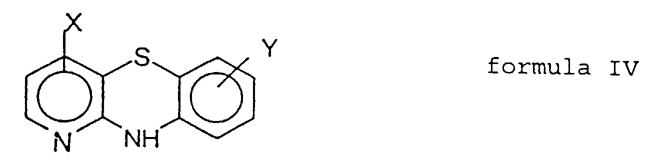 wherein X may mean hydrogen, alkyl groups having a chain length of 1-6 carbon atoms, which may be substituted by one or more halogen atoms, or carboxy having 1-5 carbon atoms and wherein the stated groups may occur once or repeatedly in positions 4, 5 or 6 of the 2-chloropyridine parent substance, and wherein Y may mean hydrogen, alkyl groups having a chain length of 1-6 carbon atoms, which may be substituted by one or more halogen atoms, halogens, alkoxy having 1-5 carbon atoms or carboxy having 1-5 carbon atoms and wherein the stated groups may occur once or repeatedly in positions 4, 5 or 6 of the 2-mercaptoaniline parent substance, characterised in that compounds of the general formula 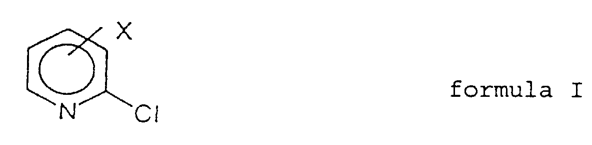 wherein X has the stated meaning,
are reacted with compounds of the general formula II 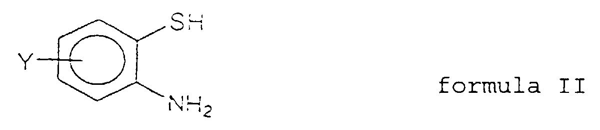 wherein Y has the stated meaning,
in inert solvents in the presence of oxidising agents.

4. Process according to claim 3, characterised in that the oxidising agents used are iodine, iron(III) salts, MnO₂, peroxides, per acids, H₂O₂, V/Mo/Ag oxides or free-radical initiators.

5. Process according to claim 3, in which X means a methyl group, ethyl group, propyl group, isopropyl group, butyl group, isobutyl group or tert.-butyl group and Y means a methyl group, ethyl group, propyl group, isopropyl group, butyl group, isobutyl group, tert.-butyl group, fluoride, chloride or bromide.

## Revendications

1. 2-(2-mercaptophénylamino)pyridine de formule V

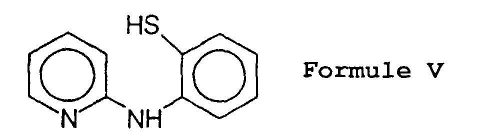

2. Procédé de fabrication de 2-(2-mercaptophénylamino)pyridine de formule V 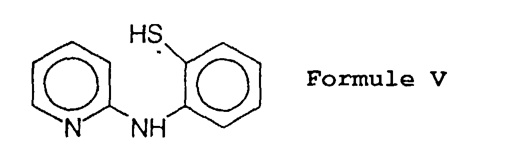 caractérisé en ce qu'
on fait réagir un composé de formule générale I, 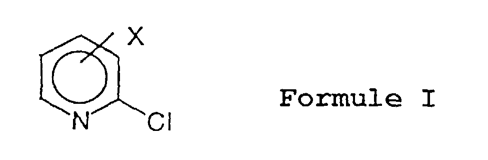 dans laquelle X représente un hydrogène, avec un composé de formule générale II, 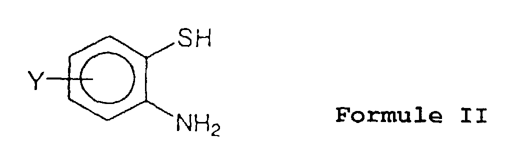 dans laquelle Y représente un hydrogène, pour donner un composé selon la revendication 1.

3. Procédé de fabrication d'azaphénothiazines de formule générale IV, 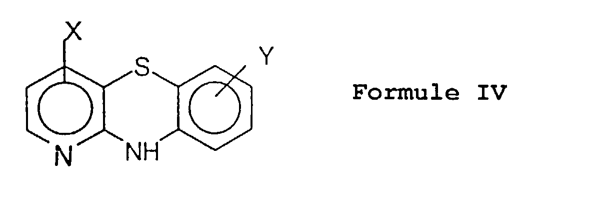 dans laquelle X représente un hydrogène, les groupes alkyle ayant une longueur de chaîne de 1 à 6 atomes de carbone, qui peut être substitué par un ou plusieurs atomes d'halogène, ou un carboxy ayant de 1 à 5 atomes de carbone, et/ou les groupes mentionnés peuvent se présenter une ou plusieurs fois dans les positions 4, 5 ou 6 du corps de base 2-chloropyridine, et/ou Y représente un hydrogène, des groupes alkyle ayant une longueur de chaîne ayant de 1 à 6 atomes de carbone, qui peut être substitué par un ou plusieurs atomes d'halogène, des halogènes, des alcoxy ayant de 1 à 5 atomes de carbone, ou des carboxy ayant de 1 à 5 atomes de carbone, et/ou les groupes mentionnés peuvent se présenter une ou deux fois dans les positions 4, 5 ou 6 du corps de base 2-mercaptoaniline,
caractérisé en qu'
on fait réagir des composés de formule générale I 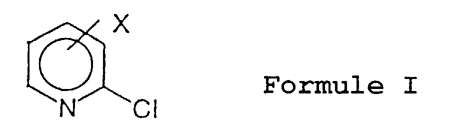 dans laquelle X a la signification mentionnée,
avec des composés de formule générale II 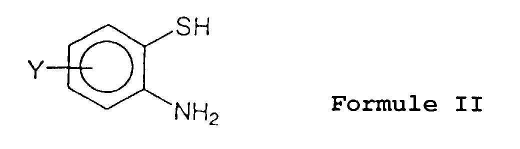 dans laquelle Y a la signification mentionnée,
en présence d'oxydants d'un des solvants inertes.

4. Procédé selon la revendication 3,
caractérisé en ce qu'
on utilise comme oxydants l'iode, les sels de fer (III), MnO₂, les peroxydes, les peracides, H₂O₂, les oxydes de V/Mo/Ag ou des initiateurs radicalaires.

5. Procédé selon la revendication 3,
dans lequel
X représente le groupe méthyle, le groupe éthyle, le groupe propyle, le groupe isopropyle, le groupe butyle, le groupe isobutyle ou le groupe tert-butyle et Y représente le groupe méthyle, le groupe éthyle, le groupe propyle, le groupe isopropyle, le groupe butyle, le groupe isobutyle, le groupe tert-butyle, un fluorure, chlorure ou bromure.
